Europäisches Patentamt

European Patent Office

Office européen des brevets

(19)

(11) Veröffentlichungsnummer: **0 004 049**
**B1**

(12) **EUROPÄISCHE PATENTSCHRIFT**

(21) Anmeldenummer: **79100640.6**

(22) Anmeldetag: **05.03.79**

(51) Int. Cl.³: **C 07 C 57/10, C 07 C 51/42**

(54) Verfahren zur Herstellung von Kaliumsorbatgranulat

(30) Priorität: **11.03.78 DE 2810702**

(43) Veröffentlichungstag der Anmeldung:
**19.09.79 Patentblatt 79/19**

(45) Bekanntmachung des Hinweises auf die Patenterteilung:
**29.10.80 Patentblatt 80/22**

(84) Benannte Vertragsstaaten:
**BE DE FR GB IT NL**

(56) Entgegenhaltungen:
**DE - A - 2 231 445**
**DE - B - 1 198 349**

(73) Patentinhaber: **HOECHST AKTIENGESELLSCHAFT**
**Zentrale Patentabteilung Postfach 80 03 20**
**D - 6230 Frankfurt Main (DE)**

(72) Erfinder: **Nöltner, Gerhard**
**Sossenheimer Weg 33a**
**D - 6230 Frankfurt Main 80 (DE)**
**Oehme, Horst, Dr.**
**Falkenstrasse 76**
**D - 6232 Bad Soden am Taunus (DE)**
**Lademann, Rudolf, Dr.**
**Im Hain 3**
**D - 6233 Kelkheim (Taunus) (DE)**
**Wendt, Heinz, Dr.**
**Kronberger Weg 20**
**D - 6231 Sulzbach (DE)**

## Verfahren zur Herstellung von Kaliumsorbatgranulat

Sorbinsäure wird zur Konservierung von Nahrungsmitteln bevorzugt in Form des in Wasser leicht löslichen Kaliumsalzes angewandt. In den letzten Jahren wird dieses Kaliumsorbat zunehmend als Granulat verarbeitet.

Es ist bekannt, daß Kaliumsorbat feuchtigkeitsempfindlich ist, leicht zusammenbackt und sich in Gegenwart von Luft infolge Zersetzung verfärbt und geruchlich verändert. Aufgrund der verminderten Oberfläche weisen Granulate eine wesentlich höhere Lagerbeständigkeit auf als das pulverförmige Produkt, das bei der Herstellung aus konzentrierter, wäßriger Lösung durch Zerstäubungstrocknung oder Kristallisation anfällt.

Für die Herstellung von Kaliumsorbatgranulat sind bisher verschiedene Verfahren beschrieben worden. Sie gehen von einer feuchten und verformbaren Paste aus, die entweder aus trockenem Kaliumsorbat durch Zumischen von Wasser (DT—OS 2617745) oder aber aus einer konzentrierten Salzlösung durch teilweises Eindampfen (DT—AS 24 50 184) hergestellt wird. Diese wird in handelsüblichen Extrudern zu Strängen gepreßt, dann geschnitten und in geeigneten Trocknern bis auf eine Restfeuchte von ca. 0,1% getrocknet.

Wegen der bereits erwähnten Zersetzungsgefahr streben die bekannten Granulierverfahren an, die Verarbeitungszeit des feuchten Kaliumsorbats so kurz wie möglich zu halten (DT—AS 24 50 184) oder zur Herstellung der granulierfähigen Paste anstelle von Wasser andere Lösemittel, wie beispielsweise niedere Alkohole, Ketone, Fettsäureester oder deren Gemische mit Wasser zu verwenden (DT—OS 26 17 745).

Da die Wasserempfindlichkeit des Kaliumsorbats mit zunehmender Temperatur steigt, sind bei den bekannten Granulierverfahren zusätzlich aufwendige Trocknungseinrichtungen erforderlich, die wegen der geringen mechanischen Festigkeit des feuchten, extrudierten Granulats äußerst schonend arbeiten müssen. Außerdem ist die für ein stabiles, lagerfähiges Produkt erforderliche Restfeuchte von weniger als 0,2% nur unter Anwendung von Vakuum zu erreichen, da sonst eine Qualitätsminderung, wie z.B. Verfärbung infolge zu hoher Temperaturen und zu langer Verweilzeiten während der Trocknung eintreten kann.

Bei der Herstellung des handelsüblichen, zylindrischen Granulats von ca. 1 mm Durchmesser durch Extrusion benötigt man eine Matrize, deren Bohrungen aber wegen ungleichmäßig verteilter Restfeuchte in der zu verformenden Masse leicht verstopfen können. Nach dem Verfahren der DT—OS 26 17 745 kann zwar eine gleichmäßige Feuchteverteilung erreicht werden man muß dabei aber erhebliche Verweilzeiten in Kauf nehmen, die wieder zu Zersetzungsverfärbungen führen können.

Bei einer partiellen Trocknung nach DT—AS 24 50 184 bis zu dem Restwassergehalt, der für eine maschinelle Extrusion erforderlich ist, weisen die flockenähnlichen Agglomerate an der Oberfläche einen wesentlich geringeren Feuchtegrad auf als im Kern.

Diese Schwierigkeiten wären zwar durch eine Trockengranulierung zu umgehen. Doch erfordern hierfür geeignete Tablettier- oder Kompaktiermaschinen sehr hohe Pressdrücke, da Bindemittel in Nahrungsmitteladditiven nicht eingesetzt werden können. Das dabei entstehende, sehr hoch verdichtete Granulat besitzt aber eine derart geringe Lösegeschwindigkeit in Wasser, daß eine praktische Anwendung, die in der Nahrungsmittelindustrie fast ausschließlich in Form der wäßrigen Lösung erfolgt, kaum möglich ist.

Es bestand daher die Aufgabe ein Granulierverfahren für Kaliumsorbat zu entwickeln, bei dem das Granulat nicht durch Zersetzungsverfärbung in der Qualität gemindert ist.

Das erfindungsgemäße Verfahren zur kontinuierlichen Herstellung eines Kaliumsorbat-Granulats ist dadurch gekennzeichnet, daß man wäßrige Kaliumsorbat-Lösung in oder auf ein durch erwärmte Luft aufgewirbeltes Bett von Kaliumsorbat-Teilchen versprüht, wobei die Menge pro Zeiteinheit an Kaliumsorbat-Lösung so eingestellt wird, daß die Temperatur des Wirbelbettes 40 bis 80°C und die relative Feuchtigkeit der das Wirbelbett verlassenden Luft höchstens 20 Gew.-%, bezogen auf 60°C, beträgt.

Die Konzentration der Kaliumsorbat-Lösung beträgt vorzugsweise mindestens 30%, besonders bevorzugt mindestens 50%. Die Obergrenze ist durch die Löslichkeit des Kaliumsorbats in Wasser gegeben, die bei 20°C etwa 58 Gew.-% beträgt.

Aus DT—OS 2.231.445 ist zwar bereits ein Verfahren zur Herstellung eines Granulats durch Aufsprühen einer Lösung oder Suspension auf ein Wirbelbett bekannt. Es werden dabei aber erhebliche Mengen an Granulat in die Wirbelschicht zurückgeführt, das an sich schon die gewünschte Korngröße hat. Eine derartige Rückführung würde bei Kaliumsorbat schnell zu einem hohen Anteil an zu großen Körnern führen, die vor der Rückführung in das Wirbelbett zerkleinert werden müßten. Eine solche Zerkleinerung beansprucht das Kaliumsorbat-Granulat thermisch. Daher muß die Menge an rückzuführendem und zu zerkleinerndem Granulat möglichst gering gehalten werden. Dies wird durch die erfindungsgemäßen Maßnahmen erreicht.

Bei dem erfindungsgemäßen Verfahren entsteht ein Kaliumsorbatgranulat, das rieselfähig ist und aus festen Teilchen besteht, die im allgemeinen kugelförmig sind. Daher ist das Granulat staubfrei oder zumindest staubarm zu handhaben und löst sich dennoch sehr leicht und schnell in Wasser.

Durch das erfindungsgemäße Verfahren wird erreicht, daß feuchtes Kaliumsorbat nur kurzfristig Temperaturen von 40—80°C ausgesetzt ist, da sich die in oder auf das Wirbelbett gesprühte wäßrige Kaliumsorbatlösung auf den Kaliumsorbatteilchen des Wirbelbettes niederschlägt und das Wasser sofort verdampft. Der Feuchtigkeitsgrad der entstehenden Granulatkörner bleibt dabei konstant unterhalb von 0,2 Gew.-%.

Bei dem erfindungsgemäßen Verfahren wird die Temperatur der zur Ausbildung des Wirbelbettes und zur Abführung des verdampften Wassers benötigten Luft so eingestellt, daß die Temperatur des Wirbelbettes 40—80°C, vorzugsweise 55—75°C, besonders bevorzugt 55—70°C beträgt. Im allgemeinen hat die Wirbelluft eine Temperatur von 100 bis 160°C, vorzugsweise 120—140°C, bevor sie mit dem Wirbelbett in Berührung kommt.

Durch die Verdampfung des als Kaliumsorbat-Lösung in das Wirbelbett eingesprühten Wassers sinkt die Temperatur der Wirbelluft, sobald sie in Kontakt mit dem Wirbelbett kommt. Gleichzeitig belädt sich die Wirbelluft mit dem verdampften Wasser. Die Menge pro Zeiteinheit an eingesprühter Kaliumsorbat-Lösung wird nun so gewählt, daß die Temperatur des Wirbelbetts und die relative Feuchtigkeit der das Wirbelbett verlassenden Luft die anspruchsgemäßen Werte erreichen. Bei hoher Luftfeuchte in der Atmosphäre ist es vorteilhaft, die Wirbelluft vor ihrer Verwendung zu trocknen. Das Einsprühen der Kaliumsorbat-Lösung kann durch Ein- oder Zweistoffdüsen erfolgen.

Bei dem erfindungsgemäßen Verfahren entfällt die bei der bekannten Granulation durch Extrusion erforderliche partielle oder vollständige Eindampfung der Kaliumsorbatlösung, mit der (ggf. nach Wiederanfeuchtung) erst ein extrudierfähiges Material hergestellt werden muß. Ebensowenig ist ein aufwendiges Nachtrocknen des fertigen Granulats notwendig. Mit dem neuen Verfahren wird also eine schnelle und dennoch schonende Herstellung eines trockenen Granulats in einem Verfahrensschritt erreicht.

Die schonende Herstellung des Kaliumsorbatgranulats bewirkt eine vernachlässigbare Zersetzungsverfärbung des Produkts, die durch Messung der Lichtdurchlässigkeit einer wäßrigen Lösung gemessen werden kann.

Im folgenden soll eine Vorrichtung (s. Figur) beschrieben werden, wie sie für das erfindungsgemäße Verfahren beispielsweise verwendet werden kann. Die Verwendung von ähnlichen Vorrichtungen ist bereits aus der oben erwähnten DT—OS 2.231.445 oder aus DT—OS 2.263.968 bekannt.

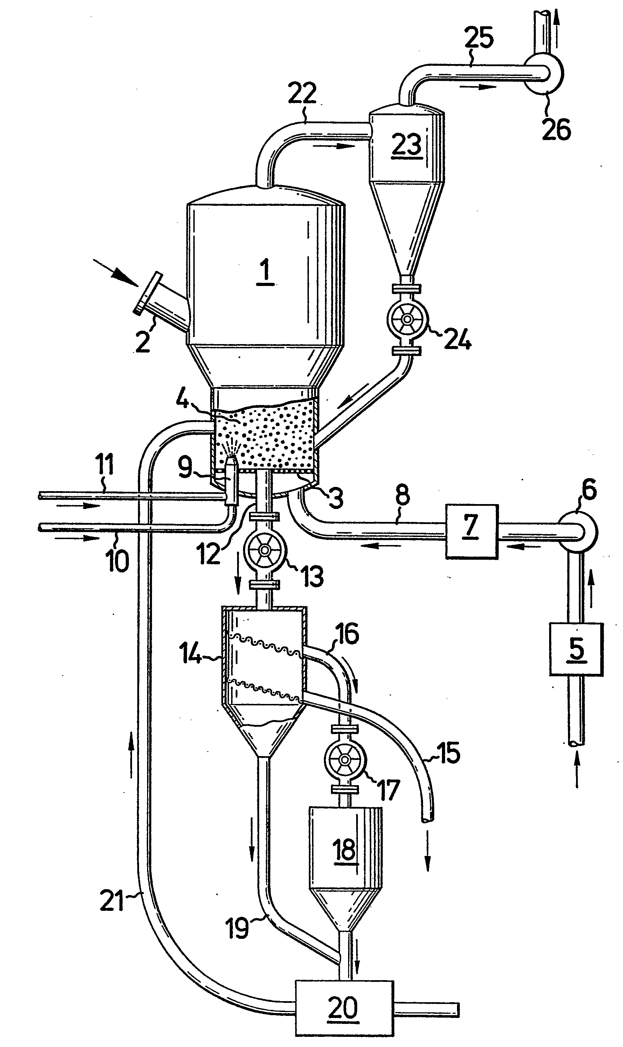

In die zylindrische Granulier- und Trocknungskammer (1) mit einem Durchmesser von 1.6 m wird über Rohr (2) trockenes Kaliumsorbat in pulvriger oder vorzugsweise granulierter Form auf den Lochboden (3) gebracht und zu einem Wirbelbett (4) aufgewirbelt. Die hierzu benutzte Wirbelluft wird über den Lufttrockner (5) mit Hilfe des Ventilators (6) aus der Atmosphäre angesaugt, im Lufterhitzer (7) erhitzt und unterhalb des waagrecht angebrachten Lochbodens (3) über Leitung (8) in die Kammer (1) eingeleitet. Gleichzeitig wird durch mehrere Zweistoffdüsen, die von unten durch die Lochplatte in das Wirbelbett hineinragen und von denen in der Figur nur eine (9) wiedergegeben ist, wäßrige Kaliumsorbat-Lösung in das Wirbelbett (4) eingesprüht. Über Leitung (10) wird den Düsen die Sorbatlösung zugeführt und über Leitung (11) Sprühluft. Die Wirbelluft tritt mit 100 bis 160°C in die Kammer (1) ein. Durch die Verdampfung des als Kaliumsorbat-Lösung in das Wirbelbett (4) eingesprühten Wassers sinkt die Temperatur der Wirbelluft, sobald sie in Kontakt mit dem Wirbelbett kommt. Durch geeignete Einstellung der Menge pro Zeiteinheit an eingesprühter Kaliumsorbat-Lösung wird die Temperatur der Wirbelluft oberhalb des Lochbodens (3) und damit die Temperatur des Wirbelbettes auf 40—80°C gebracht und die relative Feuchtigkeit der das Wirbelbett verlassenden Luft auf max. 20% (bezogen auf 60°C). Das in der Kammer (1) hergestellte Kaliumsorbat-Granulat wird über das in der Mitte des Lochbodens (3) angeordnete Rohr (12) und über die Zellenradschleuse (13) kontinuierlich ausgetragen und in der Siebvorrichtung (14) klassiert. Das Granulat der gewünschten Korngröße wird über Leitung (15) entnommen. Damit ein stationäres Wirbelbett aufrechterhalten bleibt, trägt man nur die Menge an Granulat aus, die der eingesprühten Menge an Kaliumsorbat entspricht. Die zu grobe Fraktion gelangt über Leitung (16) und Zellenrad (17) in die Mühle (18), wo sie zerkleinert wird. Das zerkleinerte Material wird ebenso wie die zu feine Fraktion (über Leitung (19)) in die pneumatische Förderung (20) gebracht und von dort über Leitung (21) zurück in die Kammer (1) transportiert. Die Wirbelluft gelangt aus der Kammer (1) über Leitung (22) in einen Zyklon (23), in dem mitgerissenes Kaliumsorbat abgeschieden wird, welches dann über ein Zellenrad (24) in die Kammer (1) zurückgeführt wird. Die im Zyklon (23) gereinigte Wirbelluft gelangt über Leitung (25) und den Ventilator (26) ins Freie.

Das nach dem erfindungsgemäßen Verfahren hergestellte Kaliumsorbatgranulat zeichnet sich dadurch aus, daß es leicht zu handhaben ist. Aufgrund seiner im allgemeinen kugelförmigen Struktur mit einem Kugeldurchmesser von etwa 0,5—1,5 mm ist es gut rieselfähig. Es besitzt eine sehr gute Abriebfestigkeit und ist daher praktisch staubfrei. Somit ist es dem durch Extrusion hergestellten Granulat überlegen, das wegen seiner zylindrischen Form viel stärker einem Abrieb unterworfen ist. Außerdem verläuft schon die erfindungsgemäße Herstellung des Granulats ohne wesentlichen Abrieb, im Unterschied zur Granulatherstellung durch Extrusion.

Die folgenden Beispiele dienen zur weiteren Erläuterung des Verfahrens.

### Beispiel 1

In der Granulier- und Trocknungskammer (1) werden 1.200 kg körniges Kaliumsorbat als Wirbelbett vorgelegt. Unterhalb des Lochbodens (3) werden 10.000 Nm³/h Luft mit einer Temperatur von 135°C und mit einem Wassergehalt von 7 g pro kg trockene Luft (entsprechend einer relativen Feuchte von 48%, bezogen auf 20°C) eingeleitet. Gleichzeitig wird über 10 kreisförmig angeordnete, durch den Lochboden (3) ragende Düsen (9) mittels auf ca. 1,3 bar verdichteter Sprühluft (1000 nm³/h) eine 50%ige Kaliumsorbatlösung (475 l/h = 500 kg/h) in das Wirbelbett (4) versprüht, so daß sich eine Bettemperatur von 65°C einstellt. Die in der Kaliumsorbatlösung enthaltene und verdampfende Wassermenge von 250 kg/h ergibt in der das Wirbelbett verlassenden Luft einen Wassergehalt von ca. 25,4 g pro kg trockener Luft, entsprechend einer relativen Luftfeuchte von weniger als 20% bezogen auf 60°C. Das gebildete Granulat mit einem Schüttgewicht von 740 g/l und einer Restfeuchte von 0,1% wird kontinuierlich über Rohr (12) abgezogen und in der Siebvorrichtung (14) klassiert. Im Mittel erhält man folgende Korngrößen-Zusammensetzung:

| 10 Gew.-% | >1,6 mm |
| 60 Gew.-% | 1,0—1,6 mm |
| 30 Gew.-% | 0,5—1,0 mm |

Der unbedeutende Feinanteil <0,5 mm (weniger als 1%) und der Grobanteil >1,6 mm (10 Gew.-%) werden, letzterer nach Passieren der Zerkleinerungsvorrichtung (18), in den unteren Teil des Granulator-Wirbelbettes, also in Düsennähe, zurückgegeben. Ebenso verfährt man mit dem Staubanteil, der aus der Abluft in einem Zyklon abgeschieden wird, bevor diese über einen Abluftventilator die Apparatur verläßt.

Verfärbungstest:

In einem Photometer wird bei einer Wellenlänge von 400 nm die Lichtdurchlässigkeit von drei Proben gemessen, wobei zum Vergleich reines Wasser (100% Lichtdurchlässigkeit)dient:

1. eingesetzte 50%ige Kaliumsorbatlösung: 96,5% Lichtdurchlässigkeit
2. 12%ige wäßrige Lösung der Granulatfraktion 0,5—1,5 mm: 95,0% Lichtdurchlässigkeit
3. 12%ige wäßrige Lösung einer Granulatfraktion 0,5—1,5 mm, die 6 Monate bei 25°C gelagert worden war: 94,0% Lichtdurchlässigkeit.

Löslichkeitstest:

In einem Becherglas wird 25 g Granulatfraktion 0,5—1,5 mm mit 100 ml entsalztem Wasser gemischt und die Zeit bis zur restlosen Auflösung des Granulats gemessen:
1. ohne Rührer 100 sec.
2. mit Rührer 11 sec.

Vergleichsbeispiel 1

Man verfährt wie im Beispiel 1, verwendet jedoch zur Ausbildung des Wirbelbetts Luft (Wirbelluft) mit einem Wassergehalt von 11 g pro kg trockener Luft, was einer relativen Luftfeuchte von 75% bezogen auf 20°C entspricht. Bei gleicher Bettemperatur von 65°C und gleicher Durchsatzmenge an 50%iger Kaliumsorbatlösung beträgt die relative Feuchte der das Wirbelbett verlassenden Luft 23%, bezogen auf 60°C, entsprechend einem Wassergehalt von 29 g pro kg trockener Luft. Men erhält ein gegenüber Beispiel 1 wesentlich gröberes Granulat mit einem mittleren Schüttgewicht von 710 g/Liter und folgender Korngrößen-Zusammensetzung:

| 90% | >1,6 mm |
| 6% | 1,0—1,6 mm |
| 4% | 0,5—1,0 mm |

Die Restfeuchte im Kornanteil >1,6 mm beträgt im Mittel 0,25%. Die (teilweise bis zu 5 mm großen) kugelförmigen Teilchen >1,6 mm ergeben beim Verfärbungstest (siehe Beispiel 1) in 12%iger wäßriger Lösung zunächst eine Lichtdurchlässigkeit von 95%. Nach einer Lagerzeit von 6 Monaten aber ist das Granulat gelbbraun verfärbt und die Lichtdurchlässigkeit beträgt nur noch 76%.

Löslichkeitstest (wie in Beispiel 1):
1. ohne Rührer 700 sec.
2. mit Rührer 24 sec.

Das heißt, das Granulat ist wesentlich schlechter löslich als das gemäß Beispiel 1 hergestellte.

Vergleichsbeispiel 2

Kaliumsorbatpulver mit einer Restfeuchte von 0,1%, das man aus 50%iger wäßriger Kaliumsorbatlösung durch Sprühtrocknung erhält, wird in einem Schneckenmischtrog mit 8% Wasser, bezogen auf Kaliumsorbat, zu einer Paste gemischt. In einer Extrusionsvorrichtung wird diese Paste mittels einer Matrize mit Bohrungen von 1,3 mm Durchmesser zu Strängen gepreßt, diese werden in ca. 5 mm lange, zylindrische Teilchen zerschnitten. Anschließend erfolgt eine Vortrocknung mit Warmluft von 53°C. Nach 45 Minuten ist eine Restfeuchte von 3% erreicht, die eine mechanische Stabilität des Granulats gewährleistet, die für eine kontinuierliche Trocknung in einem handelsüblichen Vakuumtrockner ausreicht. Nach 90 Minuten Trocknungszeit bei 65°C im Vakuum ist die erforderliche Restfeuchte von 0,1% im Granulat erreicht. Das durchschnittliche Schüttgewicht des Granulats beträgt 540 g/Liter.

Verfärbungstest (wie in Beispiel 1)

Lichtdurchlässigkeit

1. eingesetzte 50%ige Kaliumsorbatlösung: 96,5%

2. 12%ige wäßrige Lösung des durch Sprühtrocknung erhaltenen Kaliumsorbatpulvers: 95,0%

3. 12%ige wäßrige Lösung des Granulats: 96,0%

4. 12%ige wäßrige Lösung des 6 Monate bei 25°C gelagerten Granulats: 86,0%

Löslichkeitstest des Granulats (wie in Beispiel 1):
1. ohne Rührer 50 sec.
2. mit Rührer 10 sec.

## Patentansprüche

1. Verfahren zur kontinuierlichen Herstellung eines Kaliumsorbat-Granulates, dadurch gekennzeichnet, daß man wäßrige Kaliumsorbat-Lösung in oder auf ein durch erwärmte Luft aufgewirbeltes Bett von Kaliumsorbat-Teilchen versprüht, wobei die Menge pro Zeiteinheit an Kaliumsorbat-Lösung so eingestellt wird, daß die Temperatur des Wirbelbettes 40 bis 80°C und die relative Feuchtigkeit der das Wirbelbett verlassenden Luft höchstens 20 Gew.-%, bezogen auf 60°C, beträgt.

2. Verfahren nach Anspruch 1, dadurch gekennzeichnet, daß die Temperatur des Wirbelbettes 55 bis 75°C beträgt.

3. Verfahren nach Anspruch 1, dadurch gekennzeichnet, daß die Temperatur des Wirbelbettes 55 bis 70°C beträgt.

4. Verfahren nach einem der Ansprüche 1 bis 3, dadurch gekennzeichnet, daß die zum Aufwirbeln des Bettes verwendete Luft auf eine Temperatur von 100 bis 160°C erwärmt wird, ehe sie mit dem Bett in Berührung kommt.

5. Verfahren nach einem der Ansprüche 1 bis 3, dadurch gekennzeichnet, daß die zum Aufwirbeln des Bettes verwendete Luft auf eine Temperatur von 120 bis 140°C erwärmt wird, ehe sie mit dem Bett in Berührung kommt.

## Revendications

1. Procédé de fabrication en continu d'un granulé de sorbate de potassium, procédé caractérisé en ce qu'on pulvérise une solution aqueuse de sorbate de potassium dans ou sur un lit, fluidisé par de l'air chaud, de particules de sorbate de potassium, la quantité de solution de sorbate de potassium par unité de temps étant réglée de telle façon que la température du lit fluidisé soit comprise entre 40 et 80°C et que l'humidité relative de l'air quittant le lit fluidisé soit d'au plus 20% en poids, ramenée à 60°C.

2. Procédé selon la revendication 1, caractérisé en ce que la température du lit fluidisé est de 55 à 75°C.

3. Procédé selon la revendication 1, caractérisé en ce que la température du lit fluidisé est de 55 à 70°C.

4. Procédé selon l'une quelconque des revendications 1 à 3, caractérisé en ce que l'air utilisé pour la fluidisation du lit est chauffé à une température de 100 à 160°C avant d'entrer en contact avec le lit.

5. Procédé selon l'une quelconque des revendications 1 à 3, caractérisé en ce que l'air utilisé pour la fluidisation du lit est chauffé à une température de 120 à 140°C avant d'entrer en contact avec le lit.

## Claims

1. A process for the continuous manufacture of potassium sorbate, characterized by spraying an aqueous potassium sorbate solution into or onto a bed of potassium sorbate particles fluidized by heated air; the amount of potassium sorbate solution per unit of time being adjusted in such a manner that the temperature of the fluidized bed is from 40 to 80°C and the relative humidity of the air leaving the fluidized bed does not exceed 20 weight %, relative to 60°C.

2. The process as claimed in Claim 1, wherein the temperature of the fluidized bed is from 55 to 75°C.

3. The process as claimed in Claim 1, wherein the temperature of the fluidized bed is from 55 to 70°C.

4. The process as claimed in one of Claims 1 to 3, which comprises heating the air used for fluidizing the bed to a temperature of from 100 to 160°C before contacting it with the bed.

5. The process as claimed in one of Claims 1 to 3, which comprises heating the air used for fluidizing the bed to a temperature of from 120 to 140°C before contacting it with the bed.